# EUROPÄISCHE PATENTSCHRIFT

(11) **EP 3 752 043 B1**
(45) Veröffentlichungstag und Bekanntmachung des Hinweises auf die Patenterteilung: **10.05.2023**
(21) Anmeldenummer: 19712900.0
(22) Anmeldetag: 12.02.2019
(51) Int. Cl.: A61B 1/06, H01L 33/62, F21V 19/00

(54) **ULTRA-MINIATURISIERTE LICHTEMITTIERENDE EINHEIT FÜR EIN MEDIZINISCH-ENDOSKOPISCHES INSTRUMENT**
ULTRA-MINIATURIZED LIGHT-EMITTING UNIT FOR A MEDICAL ENDOSCOPIC INSTRUMENT
UNITÉ ÉLECTROLUMINESCENTE ULTRA-MINIATURISÉE DESTINÉE À UN INSTRUMENT MÉDICAL ENDOSCOPIQUE

(30) Priorität: 14.02.2018 DE 102018202241
(43) Veröffentlichungstag der Anmeldung: 23.12.2020
(73) Patentinhaber: Richard Wolf GmbH, 75438 Knittlingen (DE)
(72) Erfinder: HEIMBERGER, Rudolf, 75038 Oberderdingen (DE)
(74) Vertreter: Patentanwälte Vollmann Hemmer Lindfeld Partnerschaft mbB
(86) Internationale Anmeldenummer: PCT/DE2019/200011
(87) Internationale Veröffentlichungsnummer: WO 2019/158167

(56) Entgegenhaltungen:
- WO-A2-2006/078522
- DE-A1-102013 201 808
- DE-A1-102014 110 067
- US-A1- 2006 058 584

## Beschreibung

Die vorliegende Offenbarung betrifft eine ultra-miniaturisierte lichtemittierende Einheit für ein medizinisch-endoskopisches Instrument und ein medizinisch-endoskopisches Instrument mit solch einer ultra-miniaturisierten lichtemittierenden Einheit.

Es ist beispielweise aus der DE 10 2009 049 683 B4 bekannt, eine lichtemittierende Diode (LED) oder mehr an medizinisch-endoskopischen Instrumenten vorzusehen, um damit das Innere eines menschlichen oder tierischen Körpers von innen beleuchten oder mit Licht bestrahlen zu können. Um allerdings das Einführen eines medizinischendoskopischen Instruments möglichst minimal-invasiv zu gestalten, ist es ein dauerhaftes Bestreben, die länglichen distalen Einführabschnitte solcher Instrumente möglichst schmal und fein auszugestalten. Eine Grenze für diese Miniaturisierung stellen bislang verwendete LEDs dar, deren laterale flächige Ausdehnung meist mehr als 0,5 mm² beträgt. Ein Grund dafür ist eine über den eigentlichen LED-Chip hinausgehende flächige Ausdehnung eines Basiselements zur Anbringung eines Bonddrahtes, der den Pluspol bzw. die Anode einer Versorgungsgleichspannung mit der p-dotierten Lichtabstrahlseite des LED-Chips elektrisch verbindet. Die WO 2006/078522 A1 beschreibt LEDs für eine Vielzahl von Anwendungen. Die DE 10 2014 110 067 A1 beschreibt ein optoelektrisches Bauelement und ein Verfahren zu dessen Herstellung. Die DE 10 2013 201 808 A1 beschreibt ein LED-Beleuchtungsmodul insbesondere für medizinische Endoskope mit einer Hülse und einer L-förmigen Lasche, welche einen elektrischen Anschlusskontakt auf der lichtemittierenden Seite eines LED-Chips elektrisch kontaktiert.

Die vorliegende Offenbarung stellt dagegen eine lichtemittierende Einheit mit einer lateralen flächigen Ausdehnung bereit, die nicht über die laterale flächige Ausdehnung des eigentlichen LED-Chips hinausgeht. Es wird somit erreicht, dass die lichtemittierende Einheit ultraminiaturisiert werden kann, d. h. eine laterale flächige Ausdehnung von 0,5 mm² oder weniger hat. Dies verschiebt die Grenze für die Miniaturisierung von distalen Einführabschnitten medizinisch-endoskopischer Instrumente hin zu immer kleineren und damit weniger invasiven Durchmessern.

Gemäß einem ersten Aspekt der vorliegenden Offenbarung wird eine ultra-miniaturisierte lichtemittierende Einheit für ein medizinisch-endoskopisches Instrument bereitgestellt, wobei die lichtemittierende Einheit eine Hauptabstrahlungsrichtung definiert, und ein sich im Wesentlichen orthogonal zur Hauptabstrahlungsrichtung flächig erstreckendes Halbleiterelement mit einer in Hauptabstrahlungsrichtung gewandten Lichtabstrahlseite und einer der Hauptabstrahlungsrichtung abgewandten Kontaktseite und ein Isolierelement aufweist. Das Isolierelement weist dabei einen ersten Abschnitt und einen im Wesentlichen L-förmigen und das Halbleiterelement lateral umgreifenden zweiten Abschnitt auf, sodass ein Teil des L-förmigen zweiten Abschnitts auf der Lichtabstrahlseite des Halbleiterelements aufliegt. Ein Leiterelement ist dabei im Isolierelement eingebettet und erstreckt sich vom ersten Abschnitt des Isolierelements durch den zweiten Abschnitt des Isolierelements, wobei ein erster Kontaktbereich des Leiterelements im ersten Abschnitt des Isolierelements mit einem ersten Anschlusspol elektrisch kontaktierbar ist und ein zweiter Kontaktbereich des Leiterelements im zweiten Abschnitt des Isolierelements mit der Lichtabstrahlseite des Halbleiterelements in elektrischem Kontakt steht, und wobei die Kontaktseite des Halbleiterelements mit einem zweiten Anschlusspol elektrisch kontaktierbar ist. Das Isolierelement samt eingebettetem Leiterelement ist flexibel und als gefaltete flexible Leiterplatte ausgestaltet. Die flexible Leiterplatte weist eine erste von mindestens einer im Wesentlichen orthogonal zur Hauptabstrahlungsrichtung verlaufenden Faltkante auf, wobei mittels der ersten Faltkante die L-Form des zweiten Abschnitts des Isolierelements gebildet ist.

Mit solch einem Aufbau wird die laterale Ausdehnung der Einheit minimiert, da das im Isolierelement eingebettete Leiterelement einen Bonddraht ersetzt und daher keine über das Halbleiterelement hinausgehende flächige Ausdehnung des Basiselements benötigt wird. Das Halbleiterelement kann hierbei einen LED-Chip mit einer lateralen Flächenausdehnung von 0,25 mm² oder weniger darstellen, dessen Lichtabstrahlseite vorzugsweise p-dotiert sein kann. Die Kontaktseite des Halbleiterelements kann vorzugsweise n-dotiert und mit einer leitenden und reflektierenden Metallschicht bedampft sein. Die Hauptabstrahlungsrichtung sei hier als die Raumrichtung mit maximaler Lichtabstrahlungsintensität definiert, was in diesem Fall der Normalen auf die Lichtabstrahlseite des Halbleiterelements entspricht. Das Halbleiterelement ist vorzugsweise Gallium-basiert und kann beispielsweise Indiumgalliumnitrid (InGaN oder InₓGa₁₋ₓN) aufweisen.

Der erste Anschlusspol kann hier an einen Pluspol bzw. die Anode einer Gleichspannungsversorgungsspannung und der zweite Anschlusspol an einen entsprechenden Minuspol bzw. die Kathode angeschlossen sein. Das Halbleiterelement als Diode wird damit in Stromdurchlassrichtung, die im Wesentlichen der Hauptabstrahlungsrichtung entspricht, mit Strom durchflossen, wobei sich im Bereich des Übergangs vom n-dotierten Bereich zum p-dotierten Bereich innerhalb des Halbleiterelements Photonen bilden und als Licht abgestrahlt werden. Entgegen der Hauptabstrahlungsrichtung abgestrahltes Licht kann dabei durch eine reflektierende Metallschicht auf der Kontaktseite des Halbleiterelements im Wesentlichen in die Hauptabstrahlungsrichtung reflektiert werden.

Der erste Anschlusspol und der zweite Anschlusspol können Bestandteil der lichtemittierenden Einheit sein, müssen es aber nicht sein. In einer optionalen Ausführungsform kann der erste Anschlusspol kann von einem Außenleiter eines Koaxialkabelabschnitts und der zweite Anschlusspol von einem Innenleiter des Koaxialkabelabschnitts gebildet sein. Der Koaxialkabelabschnitt und der erste Abschnitt des Isolierelements können sich dabei im Wesentlichen orthogonal zur Kontaktseite des Halbleiterelements erstrecken, sodass der erste Kontaktbereich des Leiterelements an einer radialen Außenfläche des Außenleiters des Koaxialkabelabschnitts anlegbar und mit dieser elektrisch verbindbar ist, und wobei die Kontaktseite des Halbleiterelements mit dem Innenleiter des Koaxialkabelabschnitts elektrisch verbindbar ist. Der Koaxialkabelabschnitt kann Bestandteil der lichtemittierenden Einheit sein, wobei dann der erste Kontaktbereich des Leiterelements an einer radialen Außenfläche des Außenleiters des Koaxialkabelabschnitts anliegt und mit dieser in elektrischem Kontakt steht, und wobei die Kontaktseite des Halbleiterelements mit dem Innenleiter des Koaxialkabelabschnitts in elektrischem Kontakt steht.

In einer anderen optionalen Ausführungsform der ultra-miniaturisierten lichtemittierenden Einheit sind der erste Anschlusspol und der zweite Anschlusspol Bestandteil der lichtemittierenden Einheit. Beispielsweise kann die lichtemittierende Einheit ein sich im Wesentlichen orthogonal zur Hauptabstrahlungsrichtung flächig erstreckendes Basiselement mit dem ersten Anschlusspol und dem zweiten Anschlusspol aufweisen. Dabei kann sich der erste Abschnitt des Isolierelements im Wesentlichen orthogonal zur Hauptabstrahlungsrichtung flächig zwischen dem Halbleiterelement und dem Basiselement erstrecken und eine kontaktseitige Ausnehmung aufweisen, durch welche die Kontaktseite des Halbleiterelements mit dem zweiten Anschlusspol des Basiselements in elektrischem Kontakt steht, und wobei der erste Kontaktbereich des Leiterelements mit dem ersten Anschlusspol in elektrischem Kontakt steht.

Die kontaktseitige Ausnehmung im ersten Abschnitt des Isolierelements, durch welche die Kontaktseite des Halbleiterelements mit dem zweiten Anschlusspol des Basiselements in elektrischem Kontakt steht, kann hierbei vollständig oder nur teilweise vom ersten Abschnitt des Isolierelements lateral umgeben sein. Das bedeutet, dass die kontakseitige Ausnehmung ein Loch und/oder eine seitliche Aussparung sein kann. Die kontaktseitige Ausnehmung kann zum einen den elektrischen Kontakt zwischen dem zweiten Anschlusspol des Basiselements und der Kontaktseite des Halbleiterelements erlauben, und zum anderen eine mechanische Fixierung des Isolierelements gegen laterale Bewegungen und/oder Verdrehungen bieten.

Erfindungsgemäß ist das Isolierelement samt eingebettetem Leiterelement flexibel, wobei das Isolierelement samt eingebettetem Leiterelement als gefaltete flexible Leiterplatte ausgestaltet ist. Das Isolierelement kann beispielsweise eine Polyamid-Folie aufweisen, in die eine Leiterbahn eingebettet ist. Die flexible Leiterplatte inklusive eingebettetem Leiterelement kann sehr dünn ausgestaltet sein, etwa mit einer Dicke von 25 µm oder weniger. Dadurch kann der das Halbleiterelement lateral umgreifende zweite L-förmige Abschnitt nur eine vernachlässigbar kleine laterale Ausdehnung einnehmen. Die flexible Leiterplatte kann derart vorgefaltet sein, dass sich der zweite L-förmige Abschnitt im Wesentlichen senkrecht von einer Kante des flächigen ersten Abschnitts des Isolierelements und das Halbleiterelement lateral umgreifend erstreckt. Eine flexible Leiterplatte hat außerdem den Vorteil, dass sie außerordentlich resilient gegen Verformungen während des Zusammenbaus der Einheit ist und nicht dabei bricht oder abknickt.

Erfindungsgemäß weist die flexible Leiterplatte eine erste von mindestens einer im Wesentlichen orthogonal zur Hauptabstrahlungsrichtung verlaufende Faltkante auf, wobei mittels der ersten Faltkante die L-Form des zweiten Abschnitts des Isolierelements gebildet ist. Optional kann die flexible Leiterplatte zusätzlich eine zweite von mindestens zwei im Wesentlichen orthogonal zur Hauptabstrahlungsrichtung und parallel zueinander verlaufenden Faltkanten aufweisen, wobei die zweite Faltkante zwischen dem ersten Abschnitt des Isolierelements und dem zweiten Abschnitt des Isolierelements verläuft.

Der erste und der zweite Abschnitt des Isolierelements können dadurch eine eckige U-Form bilden, die das Halbleiterelement lateral umgreift. Dazu kann die flexible Leiterplatte an der ersten und/oder zweiten Faltkante jeweils um 90° gefaltet sein. Die quer zur Hauptabstrahlungsrichtung verlaufenden Kanten der umgriffenen Lateralseite des Halbleiterelements liegen dann vorzugsweise in den Faltkanten. Das Halbleiterelement ist dabei vorzugsweise polyedrisch und/oder quaderförmig.

Optional kann das Leiterelement im Wesentlichen U-förmig mit einem ersten Bein und einem zweiten Bein ausgestaltet sein, wobei sich das erste Bein parallel zu der Kontaktseite des Halbleiterelements zumindest teilweise durch den ersten Abschnitt des Isolierelements erstreckt, und wobei sich das zweite Bein parallel zur Lichtabstrahlseite des Halbleiterelements durch den zweiten Abschnitt des Isolierelements erstreckt. Dies erleichtert eine Kontaktierung der Beine des Leiterelements innerhalb der lateralen Ausdehnungsgrenzen des Halbleiterelements.

Optional kann das Isolierelement eine erste Kontaktöffnung für den ersten Kontaktbereich des Leiterelements und eine zweite Kontaktöffnung für den zweiten Kontaktbereich des Leiterelements aufweisen, wobei die erste Kontaktöffnung zu einer von der Kontaktseite des Halbleiterelements abgewandten Seite des ersten Abschnitts des Isolierelements hin geöffnet ist und die zweite Kontaktöffnung eine abstrahlseitige Ausnehmung durch den auf der Lichtabstrahlseite des Halbleiterelements aufliegenden Teil des L-förmigen zweiten Abschnitts des Isolierelements ist. Dadurch wird eine Kontaktierung des Leiterelements während des Zusammenbaus der Einheit besonders einfach und zuverlässig. Durch die abstrahlseitige Ausnehmung kann zum einen ein elektrischer Kontakt zwischen dem zweiten Kontaktbereich des Leiterelements und der Lichtabstrahlseite des Halbleiterelements, beispielsweise durch einen die Ausnehmung füllenden Löttropfen, hergestellt werden, und zum anderen der zweite Abschnitt des Isolierelements samt dem Leiterelement am Halbleiterelement mechanisch fixiert werden. Die abstrahlseitige Ausnehmung kann vollständig oder nur teilweise von dem auf der Lichtabstrahlseite des Halbleiterelements aufliegenden Teil des L-förmigen zweiten Abschnitts des Isolierelements lateral umgeben sein. Das bedeutet, dass die abstrahlseitige Ausnehmung ein Loch und/oder eine seitliche Aussparung sein kann.

Optional kann der zweite Kontaktbereich des Leiterelements in die zweite Kontaktöffnung des Isolierelements hineinragen, wobei ein Löttropfen die zweite Kontaktöffnung vollständig füllt und den elektrischen Kontakt zwischen dem zweiten Kontaktbereich des Leiterelements und der Lichtabstrahlseite des Halbleiterelements herstellt. Alternativ oder zusätzlich zu einem Löttropfen können sowohl der zweite Kontaktbereich des Leiterelements als auch eine Erhöhung auf der Lichtabstrahlseite des Halbleiterelements in die zweite Kontaktöffnung des Isolierelements hineinragen und so in elektrischem Kontakt stehen. Ein Kleber, vorzugsweise ein elektrisch leitender Kleber, kann statt eines Löttropfens die mechanische Fixierung gewährleisten.

Optional kann die kontaktseitige Ausnehmung des flächigen ersten Abschnitts des Isolierelements eine Schablone zur Aufnahme einer passgenau dazu korrespondierenden Kontaktfläche bilden, sodass die translatorische und/oder rotatorische Positionierung des Isolierelements eindeutig festgelegt ist. Dadurch wird ebenfalls der Zusammenbau der Einheit vereinfacht und Qualitätsmängel durch Verschiebungen, Verdrehungen, oder falschen Zusammenbau vermieden.

Optional kann die Kontaktfläche von einer Erhöhung auf der Kontaktseite des Halbleiterelements und/oder von einer Erhöhung auf einer dem Halbleiterelement zugewandten Seite des Basiselements gebildet sein. Alternativ dazu kann die lichtemittierende Einheit ferner ein zwischen dem ersten Abschnitt des Isolierelements und dem Basiselement angeordnetes und sich im Wesentlichen orthogonal zur Hauptabstrahlungsrichtung flächig erstreckendes Zwischenelement aufweisen, wobei die Kontaktfläche vom Zwischenelement gebildet ist.

Optional können der erste Anschlusspol und der zweite Anschlusspol isoliert voneinander zumindest teilweise aus einer dem Halbleiterelement zugewandten Seite des Basiselements herausragen, sodass die translatorische und/oder rotatorische Positionierung des Basiselements eindeutig festgelegt ist.

Optional können der erste Anschlusspol und der zweite Anschlusspol isoliert voneinander zumindest teilweise aus einer vom Halbleiterelement abgewandten Seite des Basiselements für den Anschluss an eine Versorgungspannung herausragen.

Gemäß einem zweiten Aspekt der vorliegenden Offenbarung wird ein medizinisch-endoskopisches Instrument bereitgestellt mit einem distalen länglichen Einführabschnitt zum minimal-invasiven Einführen in einen menschlichen oder tierischen Körper, wobei der Einführabschnitt mindestens eine zuvor beschriebene ultra-miniaturisierte lichtemittierende Einheit aufweist, um damit den Körper von innen beleuchten und/oder mit Licht bestrahlen zu können.

Optional kann dabei zumindest eine der mindestens einen ultra-miniaturisierten lichtemittierenden Einheit an einer distalen Stirnseite des Einführabschnitts angeordnet sein.

Die Offenbarung ist nachfolgend anhand von einem in den Zeichnungen dargestellten Ausführungsbeispiel näher erläutert. Es zeigen:
Fig. 1 links eine perspektivische Explosionsansicht von oben und rechts eine perspektivische Explosionsansicht von unten auf eine erste beispielhafte Ausführungsform der hierin offenbarten ultra-miniaturisierten lichtemittierenden Einheit;
Fig. 2 oben eine perspektivische Ansicht von einer Seite und unten eine perspektivische Ansicht von der anderen Seite auf eine zweite beispielhafte Ausführungsform der hierin offenbarten ultra-miniaturisierten lichtemittierenden Einheit; und
Fig. 3 eine perspektivische Seitenansicht auf eine beispielhafte Ausführungsform eines distalen Einführabschnitts des hierin offenbarten medizinisch-endoskopischen Instruments.

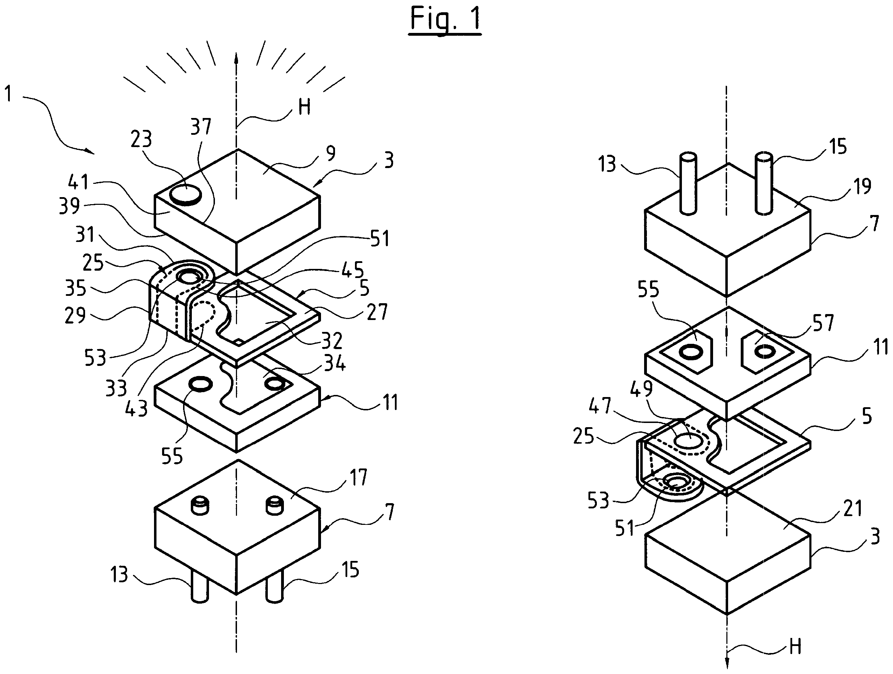

Figur 1 zeigt eine ultra-miniaturisierte lichtemittierende Einheit 1 mit einem Schichtaufbau gemäß einer ersten beispielhafte Ausführungsform mit einem flächigen Halbleiterelement 3, einem Isolierelement 5 und einem flächigen Basiselement 7, wobei das Isolierelement 5 teilweise zwischen dem Halbleiterelement 3 und dem Basiselement 7 angeordnet ist. Die Einheit 1 definiert eine Hauptabstrahlungsrichtung H, die sich im Wesentlichen senkrecht zur lateralen Ausdehnungsfläche des Halbleiterelements 3 bzw. des Basiselements 7 erstreckt. Eine vom Basiselement 7 abgewandte Seite 9 des Halbleiterelements 3 kann damit auch als Lichtabstrahlseite 9 bezeichnet werden. Zum besseren Verständnis der Fig. 1 kann hier auch von einer Lichtabstrahlseite 9 gesprochen werden, wenngleich die Einheit 1 und damit die Hauptabstrahlungsrichtung H in jedwede Raumrichtung ausgerichtet sein kann. Im Folgenden soll "oben" bzw. "über" in Richtung der Hauptabstrahlungsrichtung H bedeuten und "unten" bzw. "unter" entgegen der Hauptabstrahlungsrichtung H. Links ist in Fig. 1 demnach eine perspektivische Explosionsansicht auf die Einheit 1 von oben zu sehen und rechts eine perspektivische Explosionsansicht auf die Einheit 1 von unten. In diesem Sinne ist das Basiselement 7 im Schichtaufbau der Einheit 1 unten angeordnet und das Halbleiterelement 3 oben. Zwischen dem Isolierelement 5 und dem Basiselement 7 weist der Schichtaufbau in der in Fig. 1 gezeigten Ausführungsform ein Zwischenelement 11 auf.

Das Basiselement 7 weist zwei Anschlusspole 13, 15 auf mit einem ersten Anschlusspol 13, der als Pluspol bzw. Anode einer Gleichspannungsversorgung fungiert, und einem zweiten Anschlusspol 15, der als Minuspol bzw. Kathode der Gleichspannungsversorgung fungiert. Die Anschlusspole 13, 15 sind voneinander isoliert als sich in Hauptabstrahlungsrichtung H erstreckende Pins durch das Basiselement 7 geführt. Die Anschlusspole 13, 15 ragen sowohl auf einer dem Halbleiterelement 3 zugewandten Seite 17, sprich der Oberseite 17, des Basiselements 7 als auch auf einer vom Halbleiterelement 3 abgewandten Seite 19, sprich der Unterseite 19, des Basiselements 7 aus dem Basiselement 7. Unterseitig können die Anschlusspole 13, 15 mit der Gleichspannungsversorgung versorgt werden und oberseitig kann durch die herausragenden Anschlusspole 13, 15 ein elektrischer Kontakt in den Schichtaufbau, in diesem Fall mit dem Zwischenelement 11 erreicht werden.

Das Halbleiterelement 3 dient als eigentlicher LED-Chip und Leuchtkörper. Es ist in dieser Ausführungsform quaderförmig und im oberen Bereich p-dotiert und im unteren Bereich n-dotiert. Eine Kontaktseite 21 des Halbleiterelements 3, die hier der Unterseite 21 des Halbleiterelements 3 entspricht, ist hier mit einer elektrisch leitenden und lichtreflektierenden Metallschicht bedampft. Dadurch wird innerhalb des Halbleiterelements 3 nach unten abgestrahltes Licht nach oben reflektiert. Um das Halbleiterelement 3 zum Leuchten zu bringen, muss ein Strom am Übergang vom p-dotierten oberen Bereich in den n-dotierten unteren Bereich des Halbleiterelements 3 fließen. In einer Ecke auf der Lichtabstrahlseite 9 des Halbleiterelements 3 ragt dazu eine Kontaktfläche in Form einer Erhöhung 23 aus der Lichtabstrahlseite 9.

Das Isolierelement 5 ist hier als faltbare flexible Leiterplatte ausgestaltet mit einem eingebetteten Leiterelement 25 (gestrichelt dargestellt), das zur Kontaktierung mit der Erhöhung 23 auf der Lichtabstrahlseite 9 des Halbleiterelements 3 ausgestaltet ist. Das Isolierelement 5 weist einen ersten sich im Wesentlichen orthogonal zur Hauptabstrahlungsrichtung H flächig erstreckenden ersten Abschnitt 27 und einen im Wesentlichen L-förmigen und das Halbleiterelement 3 lateral umgreifenden zweiten Abschnitt 29 auf. In zusammengebautem Zustand der Einheit 1 liegt dabei ein oberer Teil 31 des L-förmigen zweiten Abschnitts 29 auf der Lichtabstrahlseite 9 des Halbleiterelements 3 auf. Für die Kontaktierung des zweiten Anschlusspols 15 des Basiselements 7 mit der Kontaktseite 21 des Halbleiterelements 3 weist der erste Abschnitt 27 des Isolierelements 5 eine kontaktseitige Ausnehmung 32 auf, durch welche der zweite Anschlusspol 15 in zusammengebautem Zustand der Einheit 1 mit der Kontaktseite 21 des Halbleiterelements 3 in elektrischem Kontakt steht. Der erste Abschnitt 27 des Isolierelements 5 mit der ersten Ausnehmung 32 bildet hier eine Schablone bzw. einen Rahmen zur Aufnahme einer passgenau dazu korrespondierenden und herausragenden Kontaktfläche 34 auf einer Oberseite des Zwischenelements 11, sodass die translatorische und/oder rotatorische Positionierung des Isolierelements 5 im Schichtaufbau eindeutig festgelegt ist. Die Kontaktfläche 34 des Zwischenelements 11 liegt im zusammengebauten Zustand der Einheit 1 durch die kontaktseitige Ausnehmung 32 des Isolierelements 5 hindurchgreifend elektrisch leitend an der Kontaktseite 21 des Halbleiterelements 3 an und schließt somit den Minuspol 15 an diese an.

Die Form des Isolierelements 5 wird hier durch zwei zueinander im Wesentlichen parallele und im Wesentlichen orthogonal zur Hauptabstrahlungsrichtung H verlaufende Faltkanten 33, 35 geprägt, wobei mittels einer ersten 35 der beiden Faltkanten 33, 35 die L-Form des zweiten Abschnitts 29 des Isolierelements 5 gebildet ist. Die zweite 33 der beiden Faltkanten 33, 35 verläuft zwischen dem ersten Abschnitt 27 des Isolierelements 5 und dem zweiten Abschnitt 29 des Isolierelements 5. Das Isolierelement 5 ist an den zwei Faltkanten 33, 35 jeweils um ca. 90° gefaltet, sodass sich für das Isolierelement 5 eine U-Form ergibt. Der obere auf der Lichtabstrahlseite 9 des Halbleiterelements 3 oben aufliegende Teil 31 des L-förmigen zweiten Abschnitts 29 des Isolierelements 5 verläuft daher im Wesentlichen parallel zum ersten Abschnitt 27 des Isolierelements 5. Quer zur Hauptabstrahlungsrichtung H verlaufende Kanten 37, 39 der umgriffenen Lateralseite 41 des Halbleiterelements 3 liegen dann in zusammengebautem Zustand der Einheit 1 vorzugsweise in den Faltkanten 35, 33.

Das in das Isolierelement 5 eingebettete Leiterelement 25 erstreckt sich vom ersten Abschnitt 27 des Isolierelements 5 zum Großteil durch den zweiten L-förmigen Abschnitt 29 des Isolierelements 5. Das Leiterelement 25 ist hier im Wesentlichen U-förmig mit einem ersten Bein 43 und einem zweiten Bein 45 ausgestaltet ist, wobei sich das erste Bein 43 parallel zur Kontaktseite 21 des Halbleiterelements 3 durch den ersten Abschnitt 27 des Isolierelements 5 erstreckt. Das zweite Bein 45 erstreckt sich parallel zur Lichtabstrahlseite 9 des Halbleiterelements 3 durch den oberen auf der Lichtabstrahlseite 9 des Halbleiterelements 3 aufliegenden Teil 31 des zweiten Abschnitts 29 des Isolierelements 5.

Um die elektrische Kontaktierung des eingebetteten Leiterelements 25 zu ermöglichen, weist das Isolierelement 5 eine erste untere Kontaktöffnung 47 für einen ersten unteren Kontaktbereich 49 des Leiterelements 25 und eine zweite obere Kontaktöffnung 51 für einen zweiten oberen Kontaktbereich 53 des Leiterelements 25 auf. Die erste untere Kontaktöffnung 47 ist dabei nach unten hin geöffnet, und die zweite obere Kontaktöffnung 51 ist eine abstrahlseitige Ausnehmung durch den oberen aufliegenden Teil 31 des L-förmigen zweiten Abschnitts 29 des Isolierelements 5. In zusammengebautem Zustand der Einheit 1 steht der erste untere Kontaktbereich 49 des Leiterelements 25 mit dem ersten Anschlusspol 13, d.h. dem Pluspol bzw. der Anode, des Basiselements 7 in elektrischem Kontakt. Der zweite obere Kontaktbereich 53 des Leiterelements 25 steht dann mit der Lichtabstrahlseite 9 des Halbleiterelements 3 in elektrischem Kontakt. Hier kann beispielweise ein Löttropfen und/oder ein Kleber, vorzugsweise ein elektrisch leitender Kleber, den mechanischen und elektrischen Kontakt gewährleisten.

Das Zwischenelement 11 weist zwei voneinander elektrisch isolierte Durchführungen 55, 57, auf, die im zusammengebauten Zustand der Einheit 1 unterseitig jeweils mit einem der Anschlusspole 13, 15 des Basiselements 7 in elektrischem Kontakt stehen und oberseitig mit dem ersten unteren Kontaktbereich 49 des Leiterelements 25 bzw. über die Kontaktfläche 34 mit der Kontaktseite 21 des Halbleiterelements 3 in elektrischem Kontakt stehen.

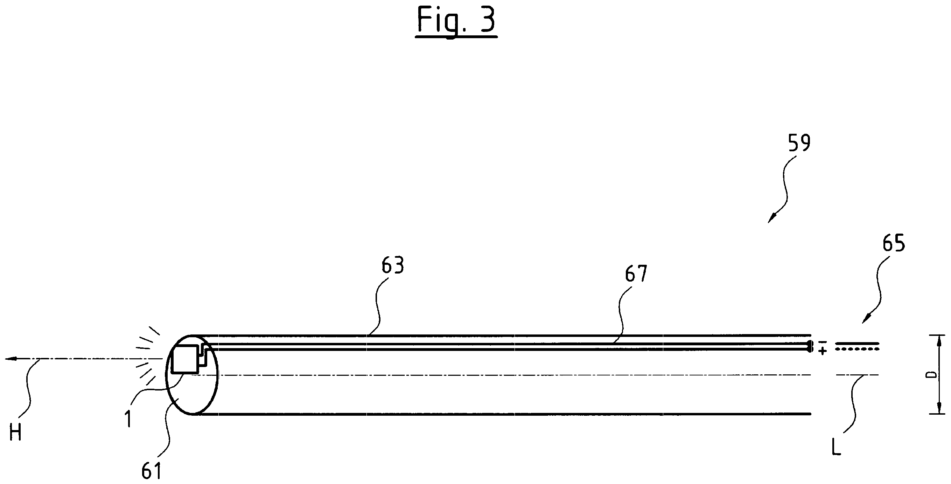

Der gesamte Schichtaufbau ist vorzugsweise zusammengepresst verklebt, um den elektrischen Kontakt zwischen den Bauteilen dauerhaft zu gewährleisten. Die laterale Ausdehnung der Einheit 1 quer zur Hauptabstrahlungsrichtung H ragt hier nur vernachlässigbar gering über die laterale Ausdehnung des LED-Chips 3 selbst hinaus. Bei einem Einbau an der Stirnseite 61 eines wie in Fig. 3 gezeigten medizinischendoskopischen Instruments 59 nimmt die Einheit 1 damit weniger Bauraum ein, sodass der Durchmesser D eines distalen Einführabschnitts 63 des minimal-invasiven Instruments 59 verringert werden kann oder dort mehr Platz für andere Funktionen zur Verfügung steht.

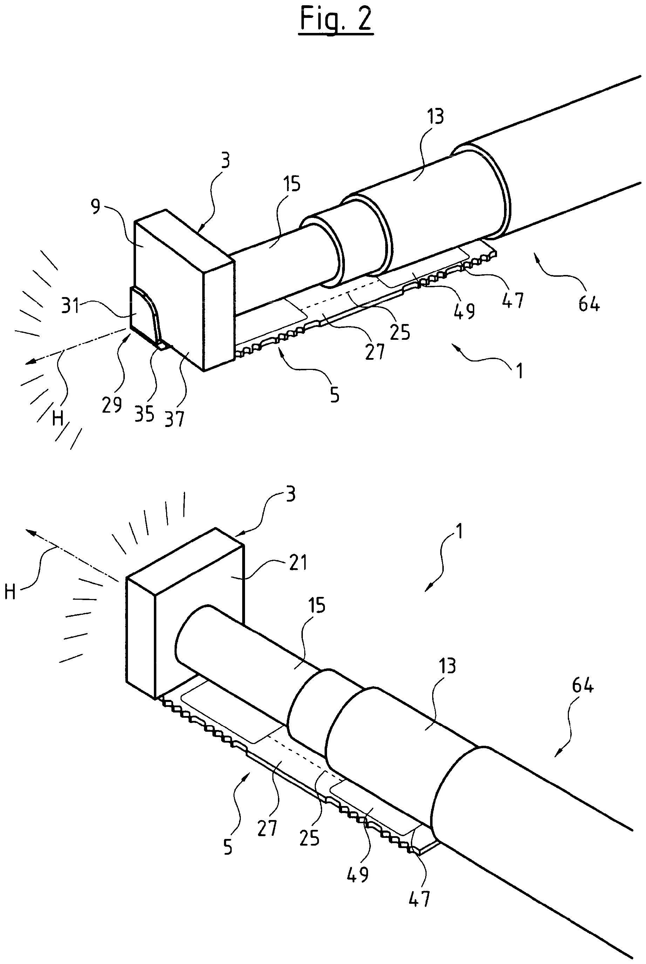

Auch in der in Fig. 2 gezeigten zweiten beispielhaften Ausführungsform ist die laterale Ausdehnung der Einheit 1 quer zur Hauptabstrahlungsrichtung H ragt auch hier nur vernachlässigbar gering über die laterale Ausdehnung des LED-Chips 3 selbst hinaus. Im Unterschied zu der in Fig. 1 gezeigten beispielhaften Ausführungsform im Schichtaufbau ist hier in der Einheit 1 kein Basiselement notwendig, das die Anschlusspole aufweist. Die Anschlusspole 13, 15 werden hier von einem Koaxialkabelabschnitt 64 gebildet, der Bestandteil der Einheit 1 sein kann oder mit dieser elektrisch verbindbar ist. Der erste Anschlusspol 13 ist dabei von einem Außenleiter des Koaxialkabelabschnitts 64 und der zweite Anschlusspol 15 von einem Innenleiter des Koaxialkabelabschnitts 64 gebildet. Der Koaxialkabelabschnitt 64 und der erste Abschnitt 27 des Isolierelements 5 erstrecken sich im Wesentlichen orthogonal zur Kontaktseite 21 des Halbleiterelements 3 bzw. parallel zur Hauptabstrahlungsrichtung H, sodass der erste Kontaktbereich 49 des Leiterelements 25 an einer radialen Außenfläche des Außenleiters 13 des Koaxialkabelabschnitts 64 anliegt und mit dieser in elektrischem Kontakt steht, und wobei die Kontaktseite 21 des Halbleiterelements 3 mit dem Innenleiter 15 des Koaxialkabelabschnitts 64 in elektrischem Kontakt steht.

Der als erster Anschlusspol 13 fungierende Außenleiter des Koaxialkabelabschnitts 64 kann mittels einer Lötverbindung und/oder Silberleitkleberverbindung mit dem ersten Kontaktbereich 49 des Leiterelements 25 sowohl mechanisch als auch elektrisch verbunden sein. Der erste Kontaktbereich 49 des Leiterelements 25 ist hier durch eine dem Koaxialkabelabschnitt 64 zugewandte erste Kontaktöffnung 47 mit dem Außenleiter des Koaxialkabelabschnitts 64 elektrisch verbunden. Der zweite Kontaktbereich 53 des Leiterelements 25 liegt analog zur Ausführungsform gemäß Fig. 1 in einer zweiten Kontaktöffnung 51, die als eine abstrahlseitige Ausnehmung durch den auf der Abstrahlseite 9 des Halbleiterelements 3 aufliegenden Teil 31 des L-förmigen zweiten Abschnitts 29 des Isolierelements 5 ausgebildet ist.

Der L-förmige zweite Abschnitt 29 des Isolierelements 5 umgreift hier wie Fig. 1 lateral das Halbleiterelement 3. In der Ausführungsform gemäß Fig. 2 sind jedoch sowohl das Isolierelement 5 als auch das Leiterelement 25 nicht U-förmig, sondern L-förmig ausgebildet. Das Isolierelement 5 weist dabei nur eine Faltkante 35 auf, wobei eine quer zur Hauptabstrahlungsrichtung H verlaufende abstrahlseitige Kante 37 der (in Fig. 2 nicht sichtbaren) umgriffenen Lateralseite 41 des Halbleiterelements 3 in der Faltkante 35 liegt. Zur mechanischen Stabilisierung kann der Koaxialkabelabschnitt 64 zwischen der ersten Kontaktöffnung 47 und dem Halbleiterelement 3 mit dem Isolierelement 5 zumindest abschnittsweise verklebt sein.

In Figur 3 ist gezeigt wie die LED-Einheit 1 gemäß der ersten und/oder zweiten beispielhaften Ausführungsform an der Stirnseite 61 angeordnet ist, sodass die Hauptabstrahlungsrichtung H im Wesentlichen parallel zur Längsachse L des Einführabschnitts 63 verläuft. Der Einführabschnitt 63 kann dabei gerade, flexibel, gekrümmt oder abknickbar ausgestaltet sein. Über die Länge des Einführabschnitts 63 kann eine proximale Gleichspannungsquelle 65 über eine Kabelführung 67 und/oder leitende Schaftteile zur LED-Einheit 1 geführt sein. Die Kabelführung 67 kann gemäß der zweiten beispielhaften Ausführungsform den Koaxialkabelabschnitt 64 aufweisen oder aus diesem gebildet sein. Alternativ oder zusätzlich zu einer Anordnung an der Stirnseite 61 können ein oder mehrere LED-Einheiten an einer lateralen Seite des Einführabschnitts 63 angeordnet sein, um einen umgebenden Hohlraum im Körper eines Menschen oder Tieres auszuleuchten oder mit Licht zu bestrahlen.

Die nummerierten Bezeichnungen der Bauteile oder Bewegungsrichtungen als "erste", "zweite", "dritte" usw. sind hierin rein willkürlich zur Unterscheidung der Bauteile oder Bewegungsrichtungen untereinander gewählt und können beliebig anders gewählt werden. Es ist damit kein Bedeutungsrang verbunden. Eine Bezeichnung eines Bauteils oder technischen Merkmals als "erstes" soll nicht dahingehend missverstanden werden, dass es ein zweites Bauteil oder technisches Merkmal dieser Art geben muss. Außerdem können etwaige Verfahrensschritte, soweit nicht explizit anders erläutert oder zwingend erforderlich, in beliebiger Reihenfolge und/oder zeitlich teilweise oder ganz überlappend durchgeführt werden.

## Patentansprüche

1. Ultra-miniaturisierte lichtemittierende Einheit (1) für ein medizinisch-endoskopisches Instrument, wobei die lichtemittierende Einheit (1) eine Hauptabstrahlungsrichtung (H) definiert, und
- ein sich im Wesentlichen orthogonal zur Hauptabstrahlungsrichtung (H) flächig erstreckendes Halbleiterelement (3) mit einer in Hauptabstrahlungsrichtung (H) gewandten Lichtabstrahlseite (9) und einer der Hauptabstrahlungsrichtung (H) abgewandten Kontaktseite (21) und
- ein Isolierelement (5) aufweist,
wobei das Isolierelement (5) einen ersten Abschnitt (27) und einen im Wesentlichen L-förmigen und das Halbleiterelement (3) lateral umgreifenden zweiten Abschnitt (29) aufweist, sodass ein Teil (31) des L-förmigen zweiten Abschnitts (29) auf der Lichtabstrahlseite (9) des Halbleiterelements (3) aufliegt,
wobei ein Leiterelement (25) im Isolierelement (5) eingebettet ist und sich vom ersten Abschnitt (27) des Isolierelements (5) durch den zweiten Abschnitt (29) des Isolierelements (5) erstreckt, wobei ein erster Kontaktbereich (49) des Leiterelements (25) im ersten Abschnitt (27) des Isolierelements (5) mit einem ersten Anschlusspol (13) elektrisch kontaktierbar ist und ein zweiter Kontaktbereich (53) des Leiterelements (25) im zweiten Abschnitt (29) des Isolierelements (5) mit der Lichtabstrahlseite (9) des Halbleiterelements (3) in elektrischem Kontakt steht,
und wobei die Kontaktseite (21) des Halbleiterelements (3) mit einem zweiten Anschlusspol (15) elektrisch kontaktierbar ist, wobei das Isolierelement (5) samt eingebettetem Leiterelement (25) flexibel und als gefaltete flexible Leiterplatte ausgestaltet ist, wobei die flexible Leiterplatte (5) eine erste von mindestens einer im Wesentlichen orthogonal zur Hauptabstrahlungsrichtung (H) verlaufenden Faltkante (35) aufweist, wobei mittels der ersten Faltkante (35) die L-Form des zweiten Abschnitts (29) des Isolierelements (5) gebildet ist.

2. Ultra-miniaturisierte lichtemittierende Einheit (1) nach Anspruch 1, wobei der erste Anschlusspol (13) von einem Außenleiter eines Koaxialkabelabschnitts und der zweite Anschlusspol (15) von einem Innenleiter des Koaxialkabelabschnitts gebildet sind, wobei sich der Koaxialkabelabschnitt und der erste Abschnitt (27) des Isolierelements (5) im Wesentlichen orthogonal zur Kontaktseite (21) des Halbleiterelements (3) erstrecken, sodass der erste Kontaktbereich (49) des Leiterelements (25) an einer radialen Außenfläche des Außenleiters des Koaxialkabelabschnitts anlegbar und mit dieser elektrisch verbindbar ist, und wobei die Kontaktseite (21) des Halbleiterelements (3) mit dem Innenleiter des Koaxialkabelabschnitts elektrisch verbindbar ist.

3. Ultra-miniaturisierte lichtemittierende Einheit (1) nach Anspruch 2, mit dem Koaxialkabelabschnitt, wobei der erste Kontaktbereich (49) des Leiterelements (25) an einer radialen Außenfläche des Außenleiters des Koaxialkabelabschnitts anliegt und mit dieser in elektrischem Kontakt steht, und wobei die Kontaktseite (21) des Halbleiterelements (3) mit dem Innenleiter des Koaxialkabelabschnitts in elektrischem Kontakt steht.

4. Ultra-miniaturisierte lichtemittierende Einheit (1) nach Anspruch 1, mit einem sich im Wesentlichen orthogonal zur Hauptabstrahlungsrichtung (H) flächig erstreckenden Basiselement (7), das den ersten Anschlusspol (13) und den zweiten Anschlusspol (15) aufweist, wobei sich der erste Abschnitt (27) des Isolierelements (5) im Wesentlichen orthogonal zur Hauptabstrahlungsrichtung (H) flächig zwischen dem Halbleiterelement (3) und dem Basiselement (7) erstreckt und eine kontaktseitige Ausnehmung (32) aufweist, durch welche die Kontaktseite (21) des Halbleiterelements (3) mit dem zweiten Anschlusspol (15) des Basiselements (7) in elektrischem Kontakt steht, und wobei der erste Kontaktbereich (49) des Leiterelements (25) mit dem ersten Anschlusspol (13) in elektrischem Kontakt steht.

5. Ultra-miniaturisierte lichtemittierende Einheit (1) nach einem der vorhergehenden Ansprüche, wobei die flexible Leiterplatte (5) eine zweite (33) von mindestens zwei im Wesentlichen orthogonal zur Hauptabstrahlungsrichtung (H) und parallel zueinander verlaufenden Faltkanten (33, 35) aufweist, wobei die zweite Faltkante (33) zwischen dem ersten Abschnitt (27) des Isolierelements (5) und dem zweiten Abschnitt (29) des Isolierelements (5) verläuft.

6. Ultra-miniaturisierte lichtemittierende Einheit (1) nach einem der vorhergehenden Ansprüche, wobei die flexible Leiterplatte (5) an der ersten Faltkante (35) und/oder an der zweiten Faltkante (33) jeweils um 90° gefaltet ist.

7. Ultra-miniaturisierte lichtemittierende Einheit nach einem der vorhergehenden Ansprüche, wobei das Leiterelement (25) im Wesentlichen U-förmig mit einem ersten Bein (43) und einem zweiten Bein (45) ausgestaltet ist, wobei sich das erste Bein (43) parallel zur Kontaktseite (21) des Halbleiterelements (3) zumindest teilweise durch den ersten Abschnitt (27) des Isolierelements (5) erstreckt, und wobei sich das zweite Bein (45) parallel zur Lichtabstrahlseite (9) des Halbleiterelements (3) durch den zweiten Abschnitt (29) des Isolierelements (5) erstreckt.

8. Ultra-miniaturisierte lichtemittierende Einheit (1) nach einem der vorhergehenden Ansprüche, wobei das Isolierelement (5) eine erste Kontaktöffnung (47) für den ersten Kontaktbereich (49) des Leiterelements (25) und eine zweite Kontaktöffnung (51) für den zweiten Kontaktbereich (53) des Leiterelements (25) aufweist, wobei die erste Kontaktöffnung (47) zu einer von der Kontaktseite (21) abgewandten Seite des ersten Abschnitts (27) des Isolierelements (5) hin geöffnet ist und die zweite Kontaktöffnung (51) eine abstrahlseitige Ausnehmung durch den auf der Lichtabstrahlseite (9) des Halbleiterelements (3) aufliegenden Teil (31) des L-förmigen zweiten Abschnitts (29) des Isolierelements (5) ist.

9. Ultra-miniaturisierte lichtemittierende Einheit nach Anspruch 8, wobei der zweite Kontaktbereich (53) des Leiterelements (25) in die zweite Kontaktöffnung (51) des Isolierelements (5) hineinragt, und wobei ein Löttropfen die zweite Kontaktöffnung (51) vollständig füllt und den elektrischen Kontakt zwischen dem zweiten Kontaktbereich (53) des Leiterelements (25) und der Lichtabstrahlseite (9) des Halbleiterelements (5) herstellt.

10. Ultra-miniaturisierte lichtemittierende Einheit nach Anspruch 8 oder 9, wobei sowohl der zweite Kontaktbereich (53) des Leiterelements (25) als auch eine Erhöhung (23) auf der Lichtabstrahlseite (9) des Halbleiterelements (3) in die zweite Kontaktöffnung (51) des Isolierelements (5) hineinragen und so in elektrischem Kontakt stehen.

11. Ultra-miniaturisierte lichtemittierende Einheit nach einem der Ansprüche 4 bis 10, wobei die kontaktseitige Ausnehmung (32) des flächigen ersten Abschnitts (29) des Isolierelements (5) eine Schablone zur Aufnahme einer passgenau dazu korrespondierenden Kontaktfläche (34) bildet, sodass die translatorische und/oder rotatorische Positionierung des Isolierelements eindeutig festgelegt ist.

12. Ultra-miniaturisierte lichtemittierende Einheit nach Anspruch 11, wobei die Kontaktfläche (34) von einer Erhöhung auf der Kontaktseite (21) des Halbleiterelements (3) und/oder von einer Erhöhung auf einer dem Halbleiterelement (3) zugewandten Seite (17) des Basiselements (7) gebildet ist.

13. Ultra-miniaturisierte lichtemittierende Einheit nach Anspruch 11, ferner mit einem zwischen dem ersten Abschnitt (27) des Isolierelements (5) und dem Basiselement (7) angeordneten und sich im Wesentlichen orthogonal zur Hauptabstrahlungsrichtung (H) flächig erstreckenden Zwischenelement (11), wobei die Kontaktfläche (34) vom Zwischenelement (11) gebildet ist.

14. Ultra-miniaturisierte lichtemittierende Einheit (1) nach einem der vorhergehenden Ansprüche 4 bis 13, wobei der erste Anschlusspol (13) und der zweite Anschlusspol (15) isoliert voneinander zumindest teilweise aus einer dem Halbleiterelement (3) zugewandten Seite (17) des Basiselements (7) herausragen, sodass die translatorische und/oder rotatorische Positionierung des Basiselements (7) eindeutig festgelegt ist.

15. Ultra-miniaturisierte lichtemittierende Einheit (1) nach einem der vorhergehenden Ansprüche 4 bis 14, wobei der erste Anschlusspol (13) und der zweite Anschlusspol (15) isoliert voneinander zumindest teilweise aus einer vom Halbleiterelement (3) abgewandten Seite (19) des Basiselements (7) für den Anschluss an eine Versorgungspannung herausragen.

16. Medizinisch-endoskopisches Instrument (59) mit einem distalen länglichen Einführabschnitt (63) zum minimal-invasiven Einführen in einen menschlichen oder tierischen Körper, wobei der Einführabschnitt (63) mindestens eine ultra-miniaturisierte lichtemittierende Einheit (1) nach einem der vorhergehenden Ansprüche aufweist, um damit den Körper von innen beleuchten und/oder mit Licht bestrahlen zu können.

17. Medizinisch-endoskopisches Instrument (59) nach Anspruch 16, wobei zumindest eine der mindestens einen ultra-miniaturisierten lichtemittierenden Einheit (1) an einer distalen Stirnseite (61) des Einführabschnitts (63) angeordnet ist.

## Claims

1. An ultra-miniaturized light-emitting unit (1) for a medical endoscopic instrument, wherein the light-emitting unit (1) defines a main emission direction (H), and has
- a semiconductor element (3) extending in a planar manner substantially orthogonally to the main emission direction (H) with a light emission side (9) facing in the main emission direction (H) and a contact side (21) facing away from the main emission direction (H), and
- an insulating element (5),
wherein the insulating element (5) has a first portion (27) and a substantially L-shaped second portion (29) laterally encompassing the semiconductor element (3), so that a part (31) of the L-shaped second portion (29) rests on the light emission side (9) of the semiconductor element (3),
wherein a conductor element (25) is embedded in the insulation element (5) and extends from the first portion (27) of the insulating element (5) through the second portion (29) of the insulating element (5), wherein a first contact region (49) of the conductor element (25) in the first portion (27) of the insulating element (5) can electrically contact a first connection terminal (13) and a second contact region (53) of the conductor element (25) in the second portion (29) of the insulating element (5) electrically contacts the light emission side (9) of the semiconductor element (3),
and wherein the contact side (21) of the semiconductor element (3) can electrically contact a second connection terminal (15), wherein the insulating element (5) inclusive of embedded conductor element (25) is flexible and is formed as a folded flexible printed circuit board, wherein the flexible printed circuit board (5) has a first of at least one fold line (35) running substantially orthogonally to the main emission direction (H), wherein the L-shape of the second portion (29) of the insulating element (5) is formed by means of the first fold edge (35).

2. The ultra-miniaturized light-emitting unit (1) according to claim 1, wherein the first connection terminal (13) is formed by an outer conductor of a coaxial cable portion and the second connection terminal (15) is formed by an inner conductor of the coaxial cable portion, wherein the coaxial cable portion and the first portion (27) of the insulating element (5) extend substantially orthogonally to the contact side (21) of the semiconductor element (3) so that the first contact region (49) of the conductor element (25) can be placed against a radial outer face of the outer conductor of the coaxial cable portion and is electrically connectable thereto, and wherein the contact side (21) of the semiconductor element (3) is electrically connectable to the inner conductor of the coaxial cable portion.

3. The ultra-miniaturized light-emitting unit (1) according to claim 2, with the coaxial cable portion, wherein the first contact region (49) of the conductor element (25) rests against a radial outer face of the outer conductor of the coaxial cable portion and electrically contacts it, and wherein the contact side (21) of the semiconductor element (3) electrically contacts the inner conductor of the coaxial cable portion.

4. The ultra-miniaturized light-emitting unit (1) according to claim 1, with a base element (7) which extends in a planar manner substantially orthogonally to the main emission direction (H) and which has the first connection terminal (13) and the second connection terminal (15), wherein the first portion (27) of the insulating element (5) extends in a planar manner substantially orthogonally to the main emission direction (H) between the semiconductor element (3) and the base element (7) and has a contact-side cutout (32), through which the contact side (21) of the semiconductor element (3) is electrically contacted with the second connection terminal (15) of the base element (7), and wherein the first contact region (49) of the conductor element (25) electrically contacts the first connection terminal (13).

5. The ultra-miniaturized light-emitting unit (1) according to any one of the preceding claims, wherein the flexible printed circuit board (5) has a second (33) of at least two fold edges (33, 35) running substantially orthogonally to the main emission direction (H) and parallel to one another, wherein the second fold edge (33) runs between the first portion (27) of the insulating element (5) and the second portion (29) of the insulating element (5).

6. The ultra-miniaturized light-emitting unit (1) according to any one of the preceding claims, wherein the flexible printed circuit board (5) is folded at the first fold edge (35) and/or at the second fold edge (33) in each case by 90°.

7. The ultra-miniaturized light-emitting unit according to any one of the preceding claims, wherein the conductor element (25) is substantially U-shaped with a first leg (43) and a second leg (45), wherein the first leg (43) extends parallel to the contact side (21) of the semiconductor element (3) at least partially through the first portion (27) of the insulating element (5), and wherein the second leg (45) extends parallel to the light emission side (9) of the semiconductor element (3) through the second portion (29) of the insulating element (5).

8. The ultra-miniaturized light-emitting unit (1) according to any one of the preceding claims, wherein the insulating element (5) has a first contact opening (47) for the first contact region (49) of the conductor element (25) and a second contact opening (51) for the second contact region (53) of the conductor element (25), wherein the first contact opening (47) is open towards a side of the first portion (27) of the insulating element (5) facing away from the contact side (21) and the second contact opening (51) is an emission-side cutout through the part (31) of the L-shaped second portion (29) of the insulating element (5) resting on the light emission side (9) of the semiconductor element (3).

9. The ultra-miniaturized light-emitting unit according to claim 8, wherein the second contact region (53) of the conductor element (25) protrudes into the second contact opening (51) of the insulating element (5), and wherein a solder droplet completely fills the second contact opening (51) and establishes the electrical contact between the second contact region (53) of the conductor element (25) and the light emission side (9) of the semiconductor element (5).

10. The ultra-miniaturized light-emitting unit according to claim 8 or 9, wherein both the second contact region (53) of the conductor element (25) and an elevation (23) on the light emission side (9) of the semiconductor element (3) protrude into the second contact opening (51) of the insulating element (5) and are thus in electrical contact.

11. The ultra-miniaturized light-emitting unit according to any one of claims 4 to 10, wherein the contact-side cutout (32) of the planar first portion (29) of the insulating element (5) forms a template for receiving a corresponding contact face (34) that accurately fits the cutout, so that the translatory and/or rotary positioning of the insulating element is clearly defined.

12. The ultra-miniaturized light-emitting unit according to claim 11, wherein the contact face (34) is formed by an elevation on the contact side (21) of the semiconductor element (3) and/or by an elevation on a side (17) of the base element (7) facing the semiconductor element (3).

13. The ultra-miniaturized light-emitting unit according to claim 11, further with an intermediate element (11), which is arranged between the first portion (27) of the insulating element (5) and the base element (7) and extends in a planar manner substantially orthogonally to the main emission direction (H), wherein the contact face (34) is formed by the intermediate element (11).

14. The ultra-miniaturized light-emitting unit (1) according to any one of preceding claims 4 to 13, wherein the first connection terminal (13) and the second connection terminal (15) protrude, insulated from one another, at least partially from a side (17) of the base element (7) facing the semiconductor element (3), so that the translatory and/or rotary positioning of the base element (7) is clearly defined.

15. The ultra-miniaturized light-emitting unit (1) according to any one of preceding claims 4 to 14, wherein the first connection terminal (13) and the second connection terminal (15) protrude, insulated from one another, at least partially from a side (19) of the base element (7) facing away from the semiconductor element (3) for the connection to a supply voltage.

16. A medical endoscopic instrument (59) with a distal elongate insertion portion (63) for minimally invasive insertion into a human or animal body, wherein the insertion portion (63) has at least one ultra-miniaturized light-emitting unit (1) according to any one of the preceding claims and thus can illuminate the body from the inside and/or can irradiate the body with light.

17. The medical endoscopic instrument (59) according to claim 16, wherein at least one of the at least one ultra-miniaturized light-emitting unit (1) is arranged on a distal end face (61) of the insertion portion (63).

## Revendications

1. Unité électroluminescente ultra-miniaturisée (1) pour un instrument médical endoscopique, l'unité électroluminescente (1) définissant une direction principale d'émission (H) et comprenant
- un élément semi-conducteur (3) s'étendant essentiellement perpendiculairement à la direction principale d'émission (H) et ayant un côté émission de lumière (9) orienté dans la direction principale d'émission (H) et un côté de contact (21) orienté à l'encontre de la direction principale d'émission (H) et
- un élément d'isolation (5),
l'élément d'isolation (5) comprenant une première section (27) et une deuxième section (29) qui a essentiellement une forme d'un L et entoure l'élément semi-conducteur (3) latéralement, de sorte qu'une partie (31) de la deuxième section (29) en forme d'un L est en appui sur le côté émission de lumière (9) de l'élément semi-conducteur (3),
un élément conducteur (25) étant intégré dans l'élément d'isolation (5) et passant de la première section (27) de l'élément d'isolation (5) par la deuxième section (29) de l'élément d'isolation (5), une première zone de contact (49) de l'élément conducteur (25) dans la première section (27) de l'élément d'isolation (5) pouvant être mise électriquement en contact à l'aide d'un premier pôle de raccordement (13) et une deuxième zone de contact (53) de l'élément conducteur (25) dans la deuxième section (29) de l'élément d'isolation (5) étant en contact électrique avec le côté émission de lumière (9) de l'élément semi-conducteur (3),
et le côté de contact (21) de l'élément semi-conducteur (3) pouvant être mis électriquement en contact à l'aide d'un deuxième pôle de raccordement (15), l'élément d'isolation (5) avec l'élément conducteur (25) intégré étant flexible et étant configuré comme un circuit imprimé flexible plié, le circuit imprimé (5) flexible comprenant un premier d'au moins un bord de pliage (35) s'étendant essentiellement perpendiculairement à la direction principale d'émission (H), la forme d'un L de la deuxième section (29) de l'élément d'isolation (5) étant constituée par le premier bord de pliage (35).

2. Unité électroluminescente ultra-miniaturisée (1) selon la revendication 1, le premier pôle de raccordement (13) étant constitué par un conducteur extérieur d'une section de câble coaxial et le deuxième pôle de raccordement (15) étant constitué par un conducteur intérieur de la section de câble coaxial, la section de câble coaxial et la première section (27) de l'élément d'isolation (5) s'étendant essentiellement perpendiculairement au côté de contact (21) de l'élément semi-conducteur (3) de sorte que la première zone de contact (49) de l'élément conducteur (25) puisse être mise en appui sur une surface extérieure radiale du conducteur extérieur de la section de câble coaxial et puisse être raccordée électriquement à celle-ci, le côté de contact (21) de l'élément semi-conducteur (3) pouvant être raccordé électriquement au conducteur intérieur de la section de câble coaxial.

3. Unité électroluminescente ultra-miniaturisée (1) selon la revendication 2 avec la section de câble coaxial, la première zone de contact (49) de l'élément conducteur (25) étant en appui sur une surface extérieure radiale du conducteur extérieur et étant électriquement en contact avec celle-ci, et le côté de contact (21) de l'élément semi-conducteur (3) étant électriquement en contact avec le conducteur intérieur de la section de câble coaxial.

4. Unité électroluminescente ultra-miniaturisée (1) selon la revendication 1 avec un élément de base (7) s'étendant sous la forme d'une surface essentiellement perpendiculairement à la direction principale d'émission (H), lequel élément comprend le premier pôle de raccordement (13) et le deuxième pôle de raccordement (15), la première section (27) de l'élément d'isolation (5) s'étendant sous la forme d'une surface perpendiculairement à la direction principale d'émission (H) entre l'élément semi-conducteur (3) et l'élément de base (7) et comprenant un évidement (32) du côté de contact par lequel le côté de contact (21) de l'élément semi-conducteur (3) est en contact électrique avec le deuxième pôle de raccordement (15) de l'élément de base (7) et la première zone de contact (49) de l'élément conducteur (25) étant en contact électrique avec le premier pôle de raccordement (13).

5. Unité électroluminescente ultra-miniaturisée (1) selon l'une des revendications précédentes, le circuit imprimé flexible (5) comprenant un deuxième (33) d'au moins deux bords de pliage (33, 35) s'étendant essentiellement perpendiculairement à la direction principale d'émission (H) et parallèlement l'un à l'autre, le deuxième bord de pliage (33) s'étendant entre la première section (27) de l'élément d'isolation (5) et la deuxième section (29) de l'élément d'isolation (5).

6. Unité électroluminescente ultra-miniaturisée (1) selon l'une des revendications précédentes, le circuit imprimé (5) flexible étant plié respectivement au premier bord de pliage (35) et/ou au deuxième bord de pliage (33) de 90°.

7. Unité électroluminescente ultra-miniaturisée (1) selon l'une des revendications précédentes, l'élément conducteur (25) ayant essentiellement la forme d'un U avec une première jambe (43) et une deuxième jambe (45), la première jambe (43) passant parallèlement au côté de contact (21) de l'élément semi-conducteur (3) et au moins partiellement par la première section (27) de l'élément d'isolation (5), et la deuxième jambe (45) passant parallèlement au côté d'émission de lumière (9) de l'élément semi-conducteur (3) par la deuxième section (29) de l'élément d'isolation (5).

8. Unité électroluminescente ultra-miniaturisée (1) selon l'une des revendications précédentes, l'élément d'isolation (5) comprenant une première ouverture de contact (47) pour la première zone de contact (49) de l'élément conducteur (25) et une deuxième ouverture de contact (51) pour la deuxième zone de contact (53) de l'élément conducteur (25), la première ouverture de contact (47) étant orientée vers un côté de la première section (27) de l'élément d'isolation (5) qui est à l'opposé du côté de contact (21) et la deuxième ouverture de contact (51) étant un évidement du côté émission de lumière (9) à travers la partie (31) de la deuxième section (29) en forme d'un L de l'élément d'isolation (5), qui est en appui sur le côté émission de lumière (9) de l'élément semi-conducteur (3).

9. Unité électroluminescente ultra-miniaturisée (1) selon la revendication 8, la deuxième zone de contact (53) de l'élément conducteur (25) s'étendant jusque dans la deuxième ouverture de contact (51) de l'élément d'isolation (5) et une goutte de matière de soudure remplissant entièrement la deuxième ouverture de contact (51) et établissant le contact électrique entre la deuxième zone de contact (53) de l'élément conducteur (25) et le côté émission de lumière (9) de l'élément semi-conducteur (5).

10. Unité électroluminescente ultra-miniaturisée (1) selon la revendication 8 ou 9, aussi bien la deuxième zone de contact (53) de l'élément conducteur (25) qu'une surélévation (23) du côté émission de lumière (9) de l'élément semi-conducteur (3) s'étendant jusque dans la deuxième ouverture de contact (51) de l'élément d'isolation (5) et étant ainsi en contact électrique.

11. Unité électroluminescente ultra-miniaturisée (1) selon l'une des revendications 4 à 10, l'évidement (32) du côté de contact de la première section (29) en forme de surface de l'élément d'isolation (5) formant un gabarit destiné à recevoir une surface de contact (34) correspondante précisément ajustée, de sorte que le positionnement en translation et/ou en rotation de l'élément d'isolation soit déterminé de façon univoque.

12. Unité électroluminescente ultra-miniaturisée (1) selon la revendication 11, la surface de contact (34) étant formée par une surélévation du côté de contact (21) de l'élément semi-conducteur (3) et/ou par une surélévation sur un côté (17) de l'élément de base (7), qui est orienté vers l'élément semi-conducteur (3).

13. Unité électroluminescente ultra-miniaturisée (1) selon la revendication 11 comprenant en outre un élément intermédiaire (11) disposé entre la première section (27) de l'élément d'isolation (5) et l'élément de base (7) et s'étendant à la façon d'une surface essentiellement perpendiculairement à la direction principale d'émission (H), la surface de contact (34) étant constituée par l'élément intermédiaire (11).

14. Unité électroluminescente ultra-miniaturisée (1) selon l'une des revendications précédentes 4 à 13, le premier pôle de raccordement (13) et le deuxième pôle de raccordement (15) dépassant au moins partiellement, de façon isolée l'un de l'autre, d'un côté (17) de l'élément de base (7), qui est orienté vers l'élément semi-conducteur (3), de sorte que le positionnement en translation et/ou en rotation de l'élément de base (7) soit déterminé de façon univoque.

15. Unité électroluminescente ultra-miniaturisée (1) selon l'une des revendications précédentes 4 à 14, le premier pôle de raccordement (13) et le deuxième pôle de raccordement (15) dépassant au moins partiellement, de façon isolée l'un de l'autre, d'un côté (17) de l'élément de base (7) qui est opposé à l'élément semi-conducteur (3), pour le raccordement à une tension d'alimentation.

16. Instrument médical endoscopique (59) ayant une section distale allongée d'introduction (63) pour une introduction à invasion minimale dans un corps humain ou animal, la section d'introduction (63) comprenant au moins une unité électroluminescente ultra-miniaturisée (1) selon l'une des revendications précédentes afin de pouvoir illuminer ledit corps de l'intérieur moyennant celle-ci et/ou de pouvoir projeter de la lumière sur celui-ci.

17. Instrument médical endoscopique (59) selon la revendication 16, au moins une desdites au moins une unité électroluminescente ultra-miniaturisée (1) étant disposée sur un côté frontal distal (61) de la zone d'introduction (63).
